# EUROPEAN PATENT SPECIFICATION

(11) **EP 3 322 380 B1**
(45) Date of publication and mention of the grant of the patent: **08.01.2025**
(21) Application number: 16825268.2
(22) Date of filing: 15.07.2016
(51) Int. Cl.: A61F 2/00, A61M 60/191, A61M 60/289, A61M 60/468, A61M 60/839, A61M 60/865, A61F 2/24

(54) **SELF-EXPANDING HEART ASSIST DEVICE**
SELBSTEXPANDIERENDE HERZUNTERSTÜTZUNGSVORRICHTUNG
DISPOSITIF D'ASSISTANCE CARDIAQUE AUTO-EXPANSIBLE

(30) Priority: 15.07.2015 US 201562192725 P
(43) Date of publication of application: 23.05.2018
(73) Proprietor: The Texas A&M University System, College Station, TX 77843-3369 (US); Corinnova Incorporated, Houston, TX 77030 (US)
(72) Inventor: CRISCIONE, John, C., College Station, TX 77845 (US); BOLCH, Christina, M., Houston, TX 77095 (US); LESCHINSKY, Boris, Mahwah, NJ 07430 (US)
(74) Representative: SONN Patentanwälte GmbH & Co KG
(86) International application number: PCT/US2016/042578
(87) International publication number: WO 2017/011778

(56) References cited:
- US-A- 5 713 954
- US-A1- 2009 043 152
- US-A1- 2010 081 867
- US-A1- 2011 257 461
- US-A1- 2013 102 849
- US-A1- 2013 102 849
- US-A1- 2014 194 671
- US-A1- 2015 165 104
- US-B2- 6 685 627
- US-B2- 8 192 351

## Description

### Technical Field of the Invention

The present invention relates in general to the field of heart assist devices, and more particularly, to a device for minimally invasive delivery of extra-cardiac devices.

### Background Art

Without limiting the scope of the invention, its background is described in connection with methods and devices for delivery or deployment of minimally invasive extra-cardiac devices. One treatment for patients who suffer from either a myocardial infarction or CHF is the implantation of a direct cardiac compression device. Currently, a sternotomy is the preferred method of implantation of the cardiac compression device. Sternotomy is a type of surgical procedure in which a vertical inline incision is made along the sternum, after which the sternum itself is divided, or "cracked". This procedure provides access to the heart for surgical procedures. Conventional direct cardiac compression devices, such as the Anstadt cup, require a sternotomy for implantation, which is a very painful procedure. Disadvantageously, sternotomies result in long recovery times and a high risk of infection. Further, there is a high risk of complications due to the lengthy surgery required for these unstable patients.

Current approaches to minimally invasive implantation of heart-assist devices of various types suffer the shortcoming of being relatively slow and difficult procedures, resulting in additional stress on the patient and reducing the likelihood of a favorable outcome. Current minimally invasive devices may be deployed via a system of guidewires placed between the pericardium and the heart. There is a need to insert minimally invasive assistive biotechnology apparatuses such as a direct cardiac compression device (DCCD) in a minimally invasive way without the use of guidewires.

US 2013/102849 A1 discloses a device that is supposed to aid in the positioning of a direct cardiac compression device about the heart. The compression device comprises wire loops having curved surfaces.

US 2010/081867 A1 concerns an apparatus for delivering a medical device onto a heart. The medical device includes a deflector. The deflector can comprise a number of wire form petals.

US 2011/257461 A1 concerns a ventricular function assisting device configured to be implanted in heart ventricle.

### Disclosure of the Invention

The present inventors recognized a need for minimally invasive devices to be deployed without the help of guidewires. In addition to speeding up insertion procedure, the present invention reduces the risk of guidewire entanglement to the patient.

The present invention provides a self-expanding wire framework device according to claim 1. More specific embodiments are defined by the dependent claims.

The self-expanding wire framework device comprises: a deployment tube and a self-expanding wire framework positioned inside the deployment tube and covered with a polymer film adapted to flare outwardly to encircle a portion of the heart, wherein the self-expanding wire framework comprises: a set of adjacent articulated wire loops extending from a lead edge to a hub, wherein each of the articulated wire loops of the set of articulated wire loops comprise a top segment positioned at the lead edge and that extends to a left midway bend and to a right midway bend, a left strut that extends from the left midway bend to the hub, a right strut that extends from the right midway bend to the hub, and a fixed strut attachment point on the hub to fix the position of the left strut and the right strut, wherein each of the articulated wire loops of the set of articulated wire loops is positioned to at least partially overlap an adjacent articulated wire loop, and wherein the left midway bend and right midway bend result in a tension that causes the set of articulated wire loops to engage in a circumferential flaring motion and bend outwardly as the self-expanding framework is deployed from the deployment tube to outwardly flair the self-expanding wire framework and the polymer film. The left midway bends and the right midway bends may be rounded to allow for gradual flaring as the self-expanding wire framework is deployed. The left midway bends and the right midway bends may be flattened to allow for flaring as the self-expanding wire framework is deployed. The left midway bends and the right midway bends may further comprise multiple bends to allow for immediate flaring positions as the self-expanding wire framework is deployed. The articulated wire loops may be a non-rounded wire. The set of articulated wire loops may comprises at least 2, 3, 4, 5, 6, 7, 8, 9, 10, 11, 12, 13, 14, 15, 16, 17, 18, 19, 20 or more articulated wire loops. The left strut may be connected to the right strut between the midway bend and the hub. The left strut may be intertwined with the right strut between the midway bend and the hub. The left strut and the right strut may be connected separately at the hub. Each of the articulated wire loops of the set of articulated wire loops may be positioned adjacent to an adjacent articulated wire loop. Each of the articulated wire loops of the set of articulated wire loops is positioned to at least partially overlap the adjacent articulated wire loop. The polymer film may be on the inside of the self-expanding wire framework. The polymer film may be on the outside of the self-expanding wire framework. The polymer film may be on the inside and the outside to sandwich the self-expanding wire framework. The left strut and the right strut each may comprise a bend end connected to the midway bend and a hub end, wherein the hub end is secured to the hub at a fixed strut attachment point. The device may further comprising a deployment tube having an outer surface surrounding an inner passage and a deployment aperture at one end of the inner passage, wherein the self-expanding wire framework covered with a polymer film is slidably positioned in the inner passage to slidably extend from the deployment aperture and adapted to flare outwardly to encircle a larger portion of the heart. The self-expanding wire framework is positioned inside the deployment tube and the top segments are bent, and the left midway bends and the right midway bends are straightened.

The present invention provides a self-expanding framework device adapted to facilitate the deployment of an extra-cardiac device, comprising: a deployment tube having an outer surface surrounding an inner passage and a deployment aperture at one end of the inner passage, and a self-expanding wire framework covered with a polymer film and slidably positioned in the inner passage to slidably extend from the deployment aperture and adapted to flare outwardly to encircle a larger portion of the heart, wherein the self-expanding wire framework comprises: a set of articulated wire loops extending from a lead edge to a hub, wherein each of the articulated wire loops of the set of articulated wire loops comprise a top segment positioned at the lead edge and that extends to a left midway bend and to a right midway bend, a left strut that extends from the left midway bend to the hub, a right strut that extends from the right midway bend to the hub, and a fixed strut attachment point on the hub to fix the position of the left strut and the right strut, wherein the left midway bend and right midway bend result in a tension that causes the self-expanding wire framework and the polymer film to engage in a circumferential flaring motion and bend outwardly as the self-expanding framework is deployed from the deployment tube.

The self-expanding wire framework is positioned inside the deployment tube with the top segments bent such that the left midway bends and the right midway bends are relatively straightened. The left midway bends and the right midway bends are rounded to allow for gradual flaring as the self-expanding wire framework is deployed from the deployment tube. The left midway bends and the right midway bends are flattened to allow for flaring as the self-expanding wire framework is deployed from the deployment tube. The left midway bends and the right midway bends further comprise multiple bends to allow for immediate flaring positions as the self-expanding wire framework is deployed from the deployment tube. The articulated wire loops may be a non-rounded wire. The set of articulated wire loops may comprises at least 2, 3, 4, 5, 6, 7, 8, 9, 10, 11, 12, 13, 14, 15, 16, 17, 18, 19, 20 or more articulated wire loops. The left strut may be connected to the right strut between the midway bend and the hub. The left strut may be intertwined with the right strut between the midway bend and the hub. The left strut and the right strut may be connected separately at the hub. Each of the articulated wire loops of the set of articulated wire loops may be positioned adjacent to an adjacent articulated wire loop. Each of the articulated wire loops of the set of articulated wire loops is positioned to at least partially overlap the adjacent articulated wire loop. The polymer film may be on the inside of the self-expanding wire framework. The polymer film may be on the outside of the self-expanding wire framework. The polymer film may be on the inside and the outside to sandwich the self-expanding wire framework. The left strut and the right strut each may comprise a bend end connected to the midway bend and a hub end, wherein the hub end is secured to the hub at a fixed strut attachment point. The device may further comprising a deployment tube having an outer surface surrounding an inner passage and a deployment aperture at one end of the inner passage, wherein the self-expanding wire framework covered with a polymer film is slidably positioned in the inner passage to slidably extend from the deployment aperture and adapted to flare outwardly to encircle a larger portion of the heart. The self-expanding wire framework is positioned inside the deployment tube and the top segments are bent, and the left midway bends and the right midway bends are straightened.

The present invention provides a self-expanding wire framework device comprising: a self-expanding wire framework covered with a polymer film. The polymer film may be a passive covering for the purposes of a passive device for diastolic heart failure. The passive covering may include one or more fluid chambers that are passively filled with fluid to compress the heart. The passive covering may include a port to adjust the volume of fluid in the passive chambers as desired.

In another embodiment the polymer film may include active inflation chambers to assist the heart. The device comprising a polymer film adapted to fit about the heart, wherein the polymer film is in contact with the heart; an outer film in contact with the biocompatible inner film; one or more fluid chambers formed between the biocompatible inner film and the outer film; and a fluid connection in fluid communication with the one or more fluid chambers, wherein a fluid enters the one or more fluid chambers through the fluid connection to selectively compress the heart during a heart contraction and during recoil the fluid exits the one or more fluid chambers through the fluid connection to allow the heart to decompress. The device may further comprise a pneumatic driver operably linked to the fluid connection to pressurize the biocompatible inner membrane to compress the heart and depressurize the biocompatible inner membrane to aid in filling the heart. In another embodiment the polymer film comprises a biocompatible inner film pneumatically locked to the heart to allow the inner film to pull open the heart and aid in filling of the heart.

In another embodiment the polymer film may include both an active set of chambers and a passive set of chambers. The device comprising an inner polymer film comprising one or more passive chambers adapted to fit about the heart. Each of the one or more passive chambers may be individually filled with a fixed volume of fluid. This fixed volume of fluid may be varied through a port to change the size of the one or more passive chambers and thus the fitment of the device about the heart. The device also includes an outer film in contact with the inner film; one or more fluid chambers formed between the inner film and the outer film; and a fluid connection in fluid communication with the one or more fluid chambers, wherein a fluid enters the one or more fluid chambers through the fluid connection to selectively compress the heart during a heart contraction and during recoil the fluid exits the one or more fluid chambers through the fluid connection to allow the heart to decompress. The device may further comprise a pneumatic driver operably linked to the fluid connection to pressurize the biocompatible inner membrane to compress the heart and depressurize the biocompatible inner membrane to aid in filling the heart. In another embodiment the polymer film comprises a biocompatible inner film pneumatically locked to the heart to allow the inner film to expand the heart and aid in filling of the heart.

The present disclosure provides a method for implanting DCCD device about the heart using a self-expanding framework delivery device, comprising the steps of: providing a self-expanding framework delivery device comprising a deployment tube having an outer surface surrounding an inner passage and a deployment aperture at one end of the inner passage, and a self-expanding wire framework covered with a polymer film and slidably positioned in the inner passage to slidably extend from the deployment aperture and adapted to flare outwardly to encircle a larger portion of the heart, wherein the self-expanding wire framework comprises: a set of articulated wire loops extending from a lead edge to a hub, wherein each of the articulated wire loops of the set of articulated wire loops comprise a top segment positioned at the lead edge and that extends to a left midway bend and to a right midway bend, a left strut that extends from the left midway bend to the hub, a right strut that extends from the right midway bend to the hub, and a fixed strut attachment point on the hub to fix the position of the left strut and the right strut, wherein the left midway bend and right midway bend result in a tension that causes the self-expanding wire framework and the polymer film to engage in a circumferential flaring motion and bend outwardly as the self-expanding framework is deployed from the deployment tube; inserting the deployment tube into the thoracic cavity; deploying the self-expanding wire framework covered with a polymer film from the deployment aperture; bending outwardly of the self-expanding framework at the left midway bend and right midway to circumferential flare the lead edge of the articulated wire loops about the apex of the heart; and extending the self-expanding wire framework from the deployment aperture to encircle a portion of the heart. The left midway bends and the right midway bends may be rounded to allow for gradual flaring as the self-expanding wire framework is deployed. The left midway bends and the right midway bends may be flattened to allow for flaring as the self-expanding wire framework is deployed. The left midway bends and the right midway bends may further comprise multiple bends to allow for immediate flaring positions as the self-expanding wire framework is deployed. The articulated wire loops may be a non-rounded wire. The set of articulated wire loops may comprises at least 2, 3, 4, 5, 6, 7, 8, 9, 10, 11, 12, 13, 14, 15, 16, 17, 18, 19, 20 or more articulated wire loops. The left strut may be connected to the right strut between the midway bend and the hub. The left strut may be intertwined with the right strut between the midway bend and the hub. The left strut and the right strut may be connected separately at the hub. Each of the articulated wire loops of the set of articulated wire loops may be positioned adjacent to an adjacent articulated wire loop. Each of the articulated wire loops of the set of articulated wire loops is positioned to at least partially overlap the adjacent articulated wire loop. The polymer film may be on the inside of the self-expanding wire framework. The polymer film may be on the outside of the self-expanding wire framework. The polymer film may be on the inside and the outside to sandwich the self-expanding wire framework. The left strut and the right strut each may comprise a bend end connected to the midway bend and a hub end, wherein the hub end is secured to the hub at a fixed strut attachment point. The device may further comprise a deployment tube having an outer surface surrounding an inner passage and a deployment aperture at one end of the inner passage, wherein the self-expanding wire framework covered with a polymer film is slidably positioned in the inner passage to slidably extend from the deployment aperture and adapted to flare outwardly to encircle a larger portion of the heart. The self-expanding wire framework is positioned inside the deployment tube and the top segments are bent, and the left midway bends and the right midway bends are straightened.

### Description of the Drawings

For a more complete understanding of the features and advantages of the present invention, reference is now made to the detailed description of the invention along with the accompanying figures and in which:
FIGURE 1 depicts a frontal view of an embodiment of the present invention.
FIGURES 2a and 2b depict a schematic diagram of the elements of the self-expanding wire framework.
FIGURES 3a-3e depict the present invention at successive amounts of deployment about a plastic mock heart.
FIGURES 4a-4d depict a side view of an embodiment of the present invention showing a single articulated wire loop being drawn into the deployment tube.
FIGURES 5a-5d depict a frontal view of an embodiment of the present invention showing a single articulated wire loop being drawn into the deployment tube.
FIGURES 6a-6c depict other embodiments of the present invention showing different designs of midway bends in the articulate wire loop.
FIGURES 7a-7d depict a side view of an embodiment of the present invention showing a single articulated wire loop with rounded midway bends being drawn into the deployment tube.
FIGURES 8a-8d depict a side view of an embodiment of the present invention showing a single articulated wire loop with dual midway bends being drawn into the deployment tube.

### Description of Embodiments

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

Though different devices exist today with specific indications for medium/long term support, devices that provide direct cardiac compression and aortic compression represent a significant innovation in the cardiac device industry as it can address both systolic and diastolic heart failure with a single device design.

The present invention is directed to a minimally invasive implantation apparatus and is adapted to save the lives of CHF patients and dramatically shorten their hospital stays. With the present invention, in combination with drug therapy and an exercise program, many of the patients receiving a deployed device, such as a cardiac compression device, in accordance with the present invention, could have restored cardiac function in as little as three weeks, allowing a shorter hospital stay and increased quality of life.

As noted, direct cardiac compression devices require a sternotomy for implantation. In contrast, the present invention permits at least a deployable device to be implanted quickly using a minimally invasive incision without the need for any assisting devices or guidewires. This allows patients to recover within a shorter period, resulting in a shorter hospital stays and less of a chance for infection.

As used herein the term "polymer" or 'polymer film" is use to denote a polymeric composition that is biocompatible. Non-limiting examples of suitable, biocompatible, biostable, implantable materials include but are not limited to polyetherurethane, polycarbonateurethane, silicone, polysiloxaneurethane, hydrogenated polystyrene-butadiene copolymer, ethylene-propylene and dicyclopentadiene terpolymer, and/or hydrogenated poly(styrene-butadiene) copolymer, poly(tetramethylene-ether glycol) urethanes, poly(hexamethylenecarbonate-ethylenecarbonate glycol) urethanes elastomeric polyurethane, latex, polyetherurethane, polycarbonateurethane, silicone, polysiloxaneurethane, hydrogenated polystyrene-butadiene copolymer, ethylene-propylene and dicyclopentadiene terpolymer, hydrogenated polystyrene-butadiene) copolymer, poly(tetramethylene-ether glycol) urethanes, poly(hexamethylenecarbonate-ethylenecarbonate glycol) urethanes and combinations thereof. In addition, the present invention may be reinforced with filaments, made of a biocompatible, biostable, implantable polyamide, polyimide, polyester, polypropylene, polyurethane etc. In addition, the device may be made from different materials in different regions of the device.

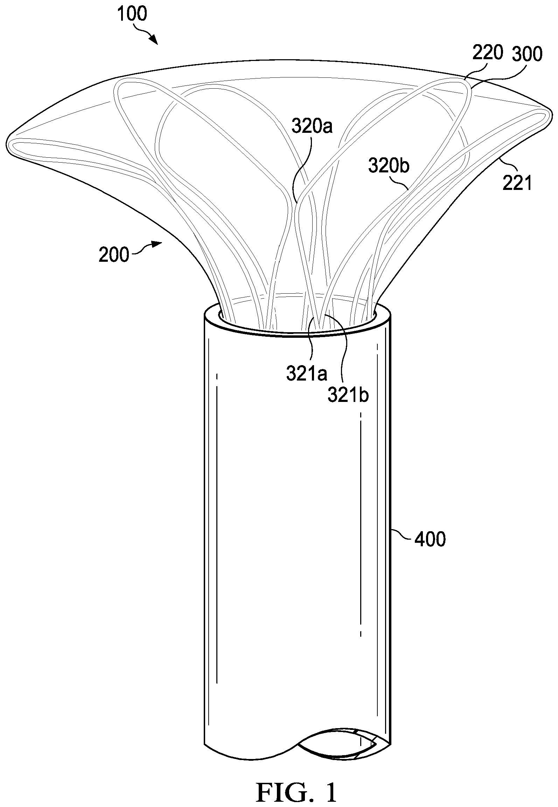

The present invention can be broadly viewed as a device comprising a deployment tube having an inner surface and an outer surface, and a self-expanding wire framework slidable along the inner surface. As shown in FIG. 1, embodiment 100 of the present invention comprises a deployment tube 400 with the self-expanding wire framework 200 having polymer film 221 and being partially deployed from the deployment tube 400. In one embodiment, the DCCD device (not shown) can be attached to the self-expanding wire framework 100 before being drawn into the deployment tube 400.

FIG. 1 shows the device 100 having a framework 200 with the polymer film 221 in contact six articulated wire loops 220 being deployed from tube 400. The leading edge or top segments 300 forms a framework 200 encloses the deployed device 100 around the heart (not shown). The self-expanding wire framework 200 comprises multiple articulated wire loops 220 adjacent each other but not intertwined and disposed in a polymer film 221. Each of the articulated wire loops 220 includes the midway bends 320a and 320b to create tension when the midway bends 320a and 320b rotate and straighten. The articulated wire loops 220 include strut 321a extending from midway bends 320a and strut 321b extending from midway bends 320b. In FIG. 1, the top segment 300 is pointed outwards or flaring, by doing so, the left and right midway bends 320a and 320b. When the top segment 300 is further deployed axially, the left and right midway bends 320a and 320b must become straighter or unbend forming a configuration with higher elastic energy. As left and right midway bends 320a and 320b of each further rotate and straighten out, this will orient the top segment 300 to straighten out from the initial flaring motion when the framework is first deployed.

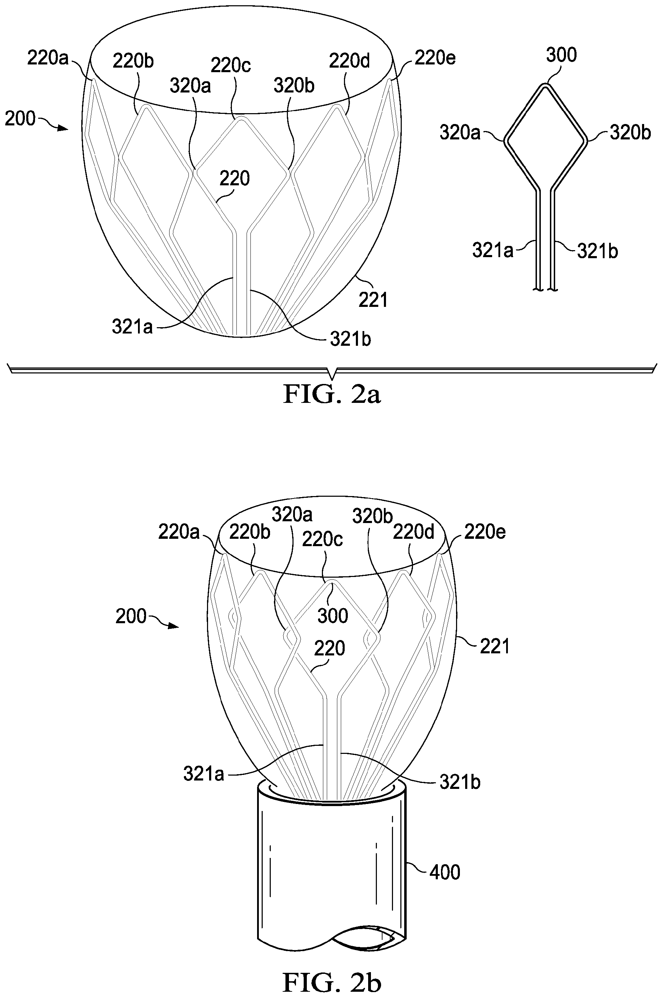

FIGS. 2a and 2b depict a schematic diagram of the elements of self-expanding wire framework 200 with a polymer film 221 covering. The self-expanding wire framework 200 is comprised of multiple articulate wire loops 220a-e, each comprising of a left and a right midway bends 320a and 320b that extend as struts 321a and 321b to hub (not shown). When the self-expanding wire framework 200 is being drawn into or compressed to fit inside the deployment tube (not shown), the top segment 300 of each articulated loops 220 is bent and compressed together to fit inside the deployment tube (not shown). The midway bends 320a and 320b of each articulated loop are straightened and compressed closer together to one another. The unbending or straightening of midway bends 320a and 320b is unstable causing it to twist or rotate rather than becoming a straight line (when struts 321a and 321b are not fixed relative to each other). When the left and right midway bends 320a and 320b of each articulated loop 220 are compressed closer together, the left and right midway bends 320a and 320b rotate and orient the top segment 300 out-of-plane creating a flaring motion. The unstable tendency of unbending makes the top segment 300 of each articulate loop 220 protrudes radially outwards away from the hoop dimension as the top segment 300 is partially deployed.

FIG. 2a shows a schematic diagram and the overall shape of the self-expanding wire framework 200 having adjacent articulated wire loops 220a-e and polymer film 221 fully deployed from the deployment tube (not shown). The leading edge or top segment 300 forms a circular framework 200 that encloses the deployed device around the heart (not shown). The self-expanding wire frame 200 comprises multiple articulated wire loops 220a-e disposed in a polymer film 221. The articulated wire loops 220a-e are adjacent each other but not intertwined. The polymer film 221 may be on one side (inner surface or outer surface) of the multiple articulated wire loops 220. The polymer film 221 may be on both sides (inner surface and outer surface) of the multiple articulated wire loops 220a-e forming a sandwich configuration. Alternatively, the polymer film 221 may be a single film with the multiple articulated wire loops 220a-e disposed within the polymer film 221. Each of the articulated wire loops 220a-e includes the midway bends 320a and 320b as shown in the diagram to create tension when the midway bends 320a and 320b rotate and straighten. The articulated wire loops 220a-e include strut 321a extending from midway bends 320a and strut 321b extending from midway bends 320b. The position of strut 321a and 321b are fixed relative each other so that the strut 321a and 321b themselves cannot rotate or turn thus forcing movement about the midway bends 320a and 320b. The left midway bend 320a and right midway bend 320b of each of the articulated wire loops 220a-e results in a tension that causes the self-expanding wire framework 200 to engage in a circumferential flaring motion and end out of plane when deployed from the deployment tube (not shown). Further deployment of the framework 200 allows the left and right midway bends 320a and 320b of each of the articulated wire loops 220a-e making up the framework 200 to straighten out in a configuration with higher elastic energy which reorients the top segment 300 back towards the center.

FIG. 2b shows a schematic diagram and the overall shape of the self-expanding wire framework 200 having overlapping articulated wire loops 220 with the polymer film 221 covering that is fully deployed from the deployment tube (not shown). The leading edge or top segment 300 forms a circular framework 200 that encloses the deployed device around the heart (not shown). The self-expanding wire framework 200 comprises multiple articulated wire loops 220 disposed in a polymer film 221 in a partially overlapping pattern with the adjacent wire loops 220 partially overlapping (but not intertwined). The polymer film 221 may be on one side (inner surface or outer surface) of the multiple articulated wire loops 220. The polymer film 221 may be on both sides (inner surface and outer surface) of the multiple articulated wire loops 220 forming a sandwich configuration. Alternatively, the polymer film 221 may be a single film with the multiple articulated wire loops 220 disposed with in the polymer film 221. Each of the articulated wire loops 220 includes the midway bends 320a and 320b as shown in the diagram to create tension when the midway bends 320a and 320b rotate and straighten. The articulated wire loops include strut 321a extending from midway bends 320a and strut 321b extending from midway bends 320b. The position of strut 321a and 321b are fixed relative to each other so that the strut 321a and 321b cannot rotate or turn forcing movement about the midway bends 320a and 320b. The left midway bend 320a and right midway bend 320b of each of the articulated wire loops 220 results in a tension that causes the self-expanding wire framework 200 to engage in a circumferential flaring motion and end out of plane when deployed from the deployment tube. Further deployment of the framework 200 allows the left and right midway bends 320a and 320b of each of the articulated wire loops 220 making up the framework 200 to straighten out in a configuration with higher elastic energy which reorients the top segment 300 back towards the center. The self-expanding wire framework 200 has partially overlapping articulated wire loops 220 which are covered by polymer film 221. Each articulated wire loop has a left midway bend 320a and a right midway bend 320b. The adjacent wire loop at least partially overlaps a second wire loop by placing the left midway bend of a first wire loop behind the right midway bend of the second wire loop this pattern is repeated until the desired number of the articulated wire loops are achieved.

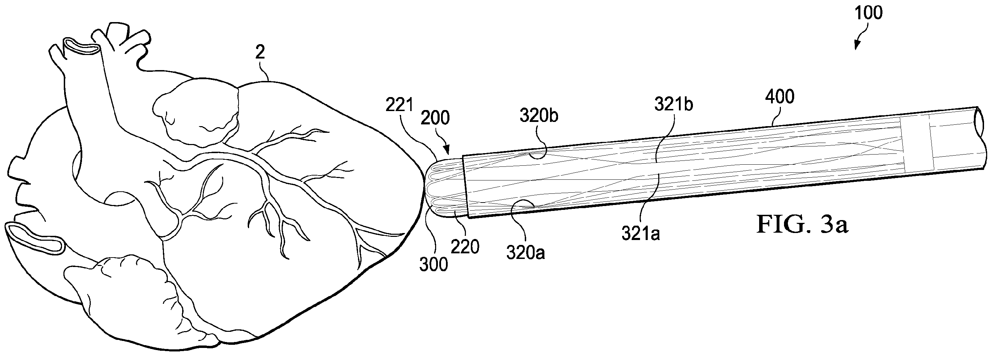

To illustrate how the self-expanding wire framework 200 able to advance around a heart 2 from an apical approach, FIGS. 3a - 3b depict an embodiment of the present invention at successive amounts of deployment about a plastic mock heart 2 (3D printed from a CT scan of a normal adult sheep).

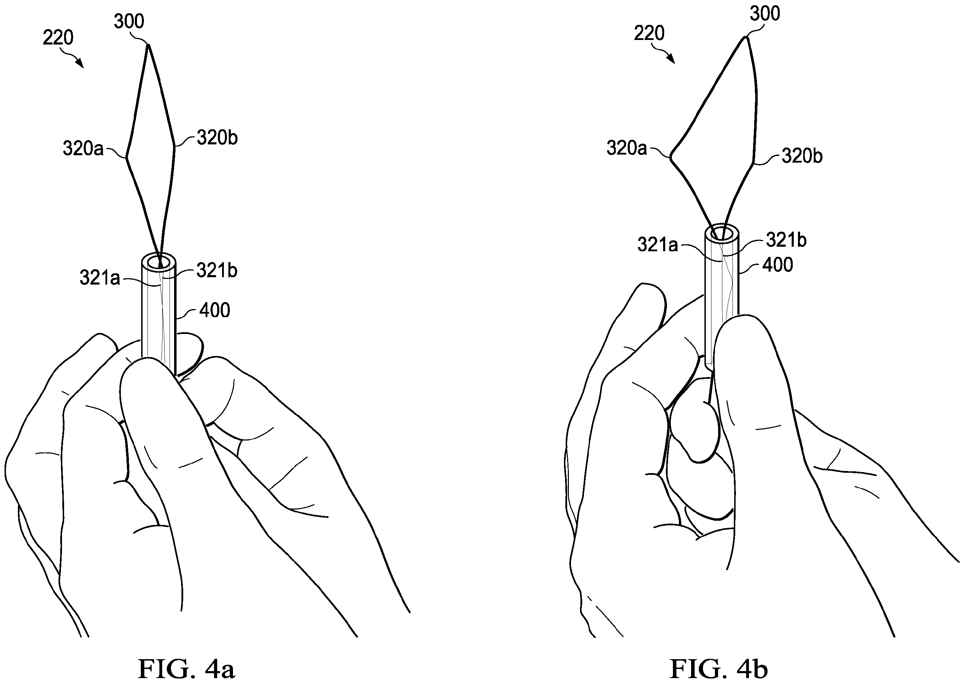

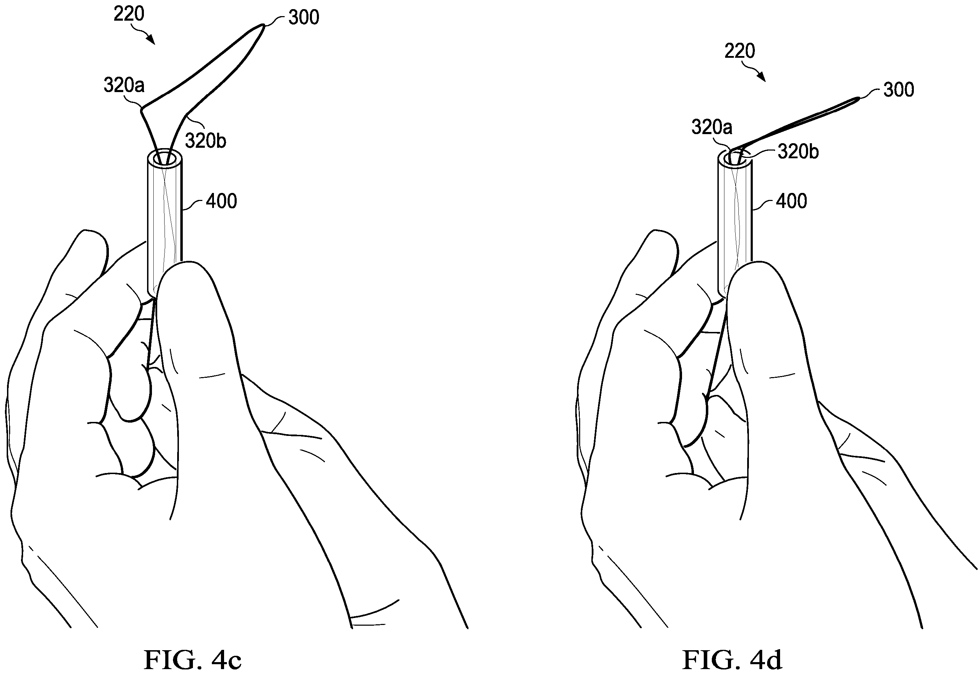

FIGS. 3a-3e illustrated the flaring and straightening of the framework 200 as it was deployed from the deployment tube 400. However, the flaring motion can also be observed with just a single articulated wire loop as seen in FIGS. 4-8.

FIG. 3a shows the self-expanding wire framework 200 compressed inside deployment tube 400 as it is deployed about a plastic mock heart 2. FIG. 3a shows a polymer film 221 with an articulated wire loop 220 with the top segment 300 flanked by the left and right midway bends 320a and 320b and include strut 321a extending from midway bends 320a and strut 321b extending from midway bends 320b as it is deployed from the deployment tube 400. The articulated wire loop 220 is deployed from the deployment tube 400 such that the left and right midway bends 320a and 320b are within the deployment tube 400.

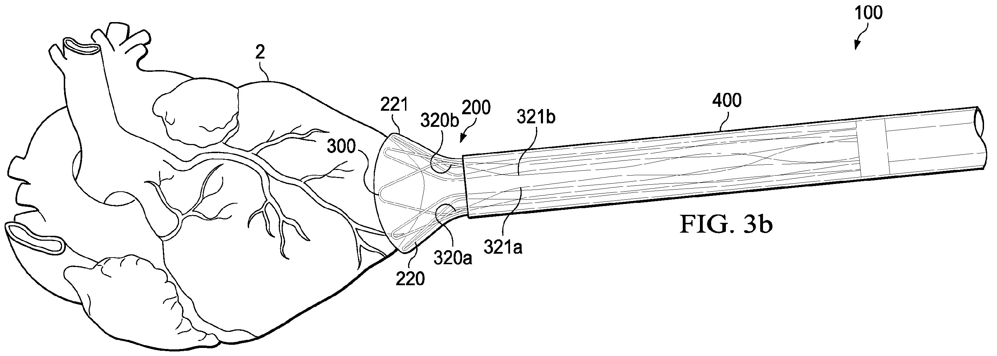

FIG. 3b shows the initial deployment of framework 200 wherein the top segments 300 of each of the articulated wire loop 220 are beginning to engage in a circumferential flaring motion and bend out of plane as it is deployed about a plastic mock heart 2. FIG. 3b shows a polymer film 221 with an articulated wire loop 220 with the top segment 300 flanked by the left and right midway bends 320a and 320b and include strut 321a extending from midway bends 320a and strut 321b extending from midway bends 320b as it is deployed from the deployment tube 400. The articulated wire loop 220 is deployed from the deployment tube 400 such that the left and right midway bends 320a and 320b are extending from the deployment tube 400.

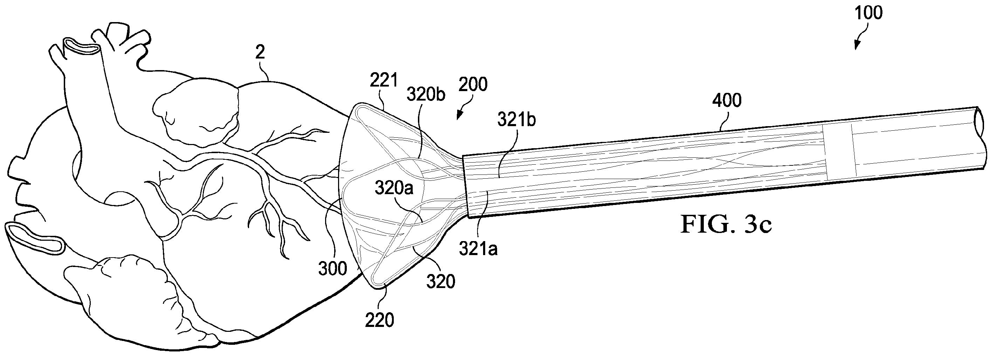

FIG. 3c illustrates the framework deployed to the midway bends 320 which are bent outwards flaring the top segment 300 out. As the framework 200 is deployed pass the midway bends 320, about a plastic mock heart 2. FIG. 3c shows a polymer film 221 with an articulated wire loop 220 with the top segment 300 flanked by the left and right midway bends 320a and 320b and include strut 321a extending from midway bends 320a and strut 321b extending from midway bends 320b as it is deployed from the deployment tube 400. The articulated wire loop 220 is deployed from the deployment tube 400 such that the left and right midway bends 320a and 320b and the polymer film 221 are extending past the deployment tube 400.

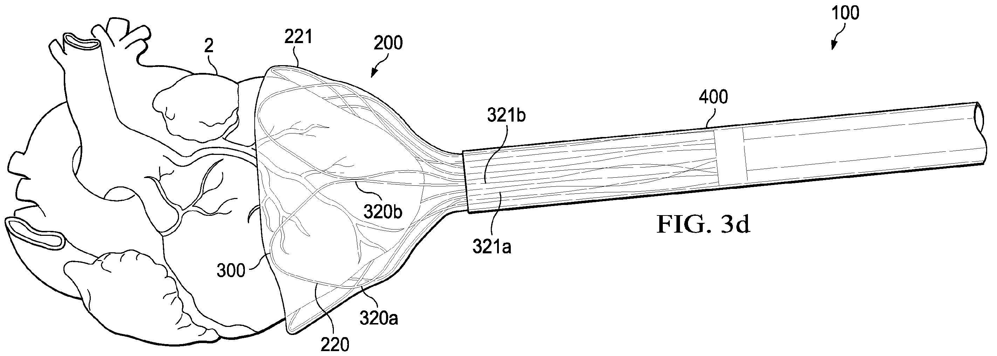

FIG. 3d shows the struts 321a and 321b connected to midway bends 320a and 320b which straighten out to configure to a form of higher elastic energy. The framework 200 is deployed pass the midway bends 320, about a plastic mock heart 2. FIG. 3d shows a polymer film 221 with an articulated wire loop 220 with the top segment 300 flanked by the left and right midway bends 320a and 320b and include strut 321a extending from midway bends 320a and strut 321b extending from midway bends 320b as it is deployed from the deployment tube 400. The articulated wire loop 220 is deployed from the deployment tube 400 such that the left and right midway bends 320a and 320b are extending past the deployment tube 400. As the midway bends 320a and 320b are straightened out, the top segment 300 of each articulated wire loop 220 are reoriented inwards from the initial flared position. The reorientation of the articulated wire loops 220 allows the top segment 300 of each wire loop 220 and the polymer film 221 to make contact with the outer circumference of the heart 2.

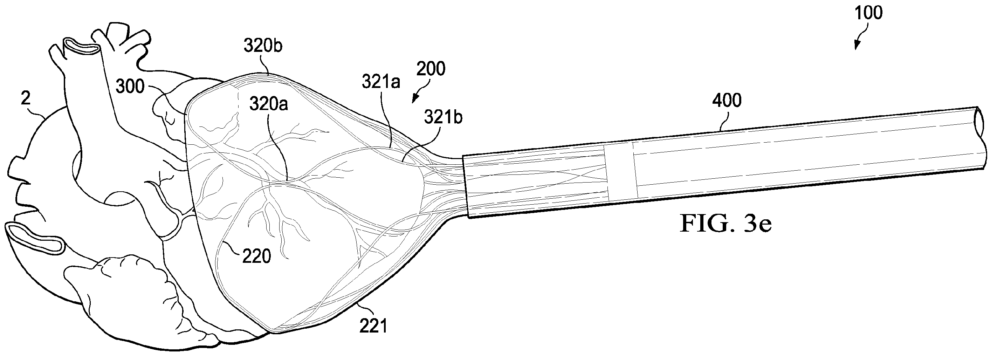

FIG. 3e illustrates the full deployment of the framework 200 as each articulated wire loop 220 is further expanded to encircle a larger portion of the heart 2. The framework 200 is deployed pass the midway bends 320, about a plastic mock heart 2. FIG. 3e shows a polymer film 221 with an articulated wire loop 220 with the top segment 300 flanked by the left and right midway bends 320a and 320b and include strut 321a extending from midway bends 320a and strut 321b extending from midway bends 320b as it is deployed from the deployment tube 400. The articulated wire loop 220 is deployed from the deployment tube 400 such that the left and right midway bends 320a and 320b and the polymer film 221 are extending past the deployment tube 400 to encircle a larger portion of the heart 2.

The self-expanding wire framework device of the present invention includes multiple articulated wire loops with a polymer film connected thereto. The number of articulated wire loops used is at the discretion of the manufacturer and depends on the size of the deployable device. As little as 2 articulated wire loops can be used, but the number can increase to 3, 4, 5, 6, 7, 8, 9, 10, 11, 12, or more articulated wire loops linked. Each of the articulated wire loops include struts that connect the midway bends to the hub (not shown). The struts are fixed in position relative to the midway bends to induce flaring when extended from the deployment tube. The fixation location can vary with the deployment of the framework as tension between the articulate wire loops may move the linkage. However, the struts between the loops may also be fixated to limit the maximum expansion size of the wire framework.

FIGS. 4a-4d depicts a side view of an embodiment of the present invention showing a single articulated wire loop 220 being drawn into the deployment tube 400. In FIG. 4a the articulated loop 220 is beginning to be drawn into the deployment tube 400 which compresses the left and right midway bends 320a and 320b together to allow top segments 300 to move inwardly. In FIG. 4b the articulated loop 220 is beginning to be drawn into the deployment tube 400 which compresses the left and right midway bends 320a and 320b together. At this point, flaring of the top segment 300 is evident between FIGS. 4a and 4b. Instead of the wire loop 220 collapsing in-plane and forced into the deployment tube 400, the left and right midway bends 320a and 320b each rotate to orient their top segments 300 out of plane. FIG. 4c shows an oriented wire loop 220 with the left and right midway bends 320a and 320b bent and flaring the top segment 300 outwardly. FIG. 4d shows the articulated wire loop 220 drawn into the deployment tube 400 to the left and right midway bends 320a and 320b. Each of the articulated wire loops 220 will have a pair of struts extending into the deployment tube 400 that may or may not be compressed. The present invention would still function if the loop ends were crossed or parallel within the deployment tube 400.

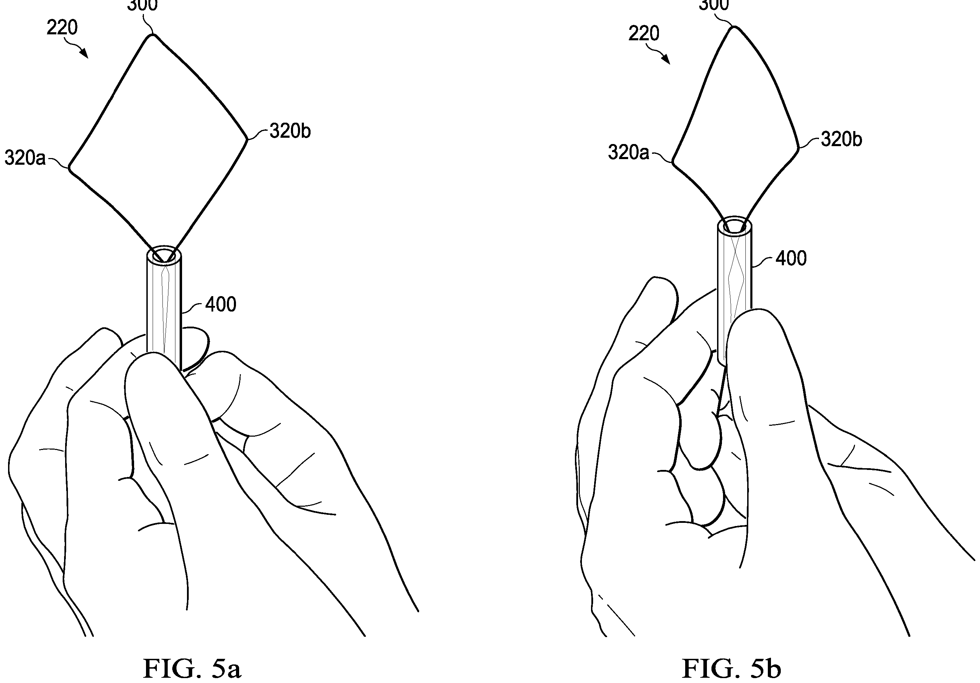

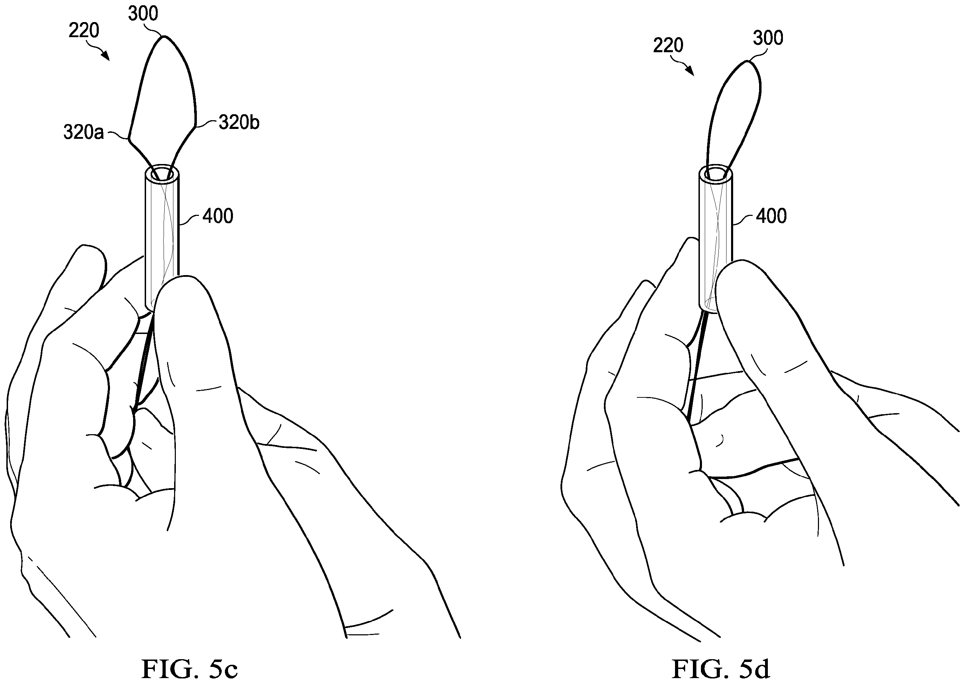

FIGS. 5a-5d depicts a frontal view of the single articulated wire loop 220 being drawn into the deployment tube 400 and correspond with FIGS. 4a-4d respectively. The bending and flaring of the midway bends 320a and 320b in the deployment tube 400 cannot be seen in the frontal view, but the compression of the midway bends 320a and 320b together is clear. FIGS. 5b and 5c show the reorientation of the wire loop 220 as the midway bends 320a and 320b are compressed into the deployment tube 400. FIG. 5d shows that after the midway bends 320a and 320b are reached, the rest of the wire loop up to the top segment 300 are compressed together to be drawn into the deployment tube 400. However, this compressed form is the configuration the loop 220 will be deployed at which leads to the initial flaring out position shown in FIG. 4d when the wire loop 220 is first deployed.

As noted herein, there are a number of features and advantages of the apparatus of the present invention, including: the apparatus is adapted to permit deployment of a deployable device, such as a heart assist or cardiac compression device; the self-expanding wire framework equipped with the deployable device being collapsible to fit inside the deployment tube to be positioned near the heart, and then deployed to advance around a heart without the need of any guidewires; the apparatus comprising a minimal number of components-including a self-expanding wire framework adapted to engage a deployable device, expand the deployable device and guide the deployable device during deployment; and the self-expanding wire framework and deployment tube being separated or integrated into a single unit.

Once the self-expanding wire framework is deployed from the deployment, controlled expansion and outward bend of the framework can aid in the positioning and implantation of the deployable device. The wire used for constructing the self-expanding wire frame will need to be an appropriate wire stiffness to achieve desired expansion and flaring out but not cause inversion of the wireframe during deployment. The use of quadrilateral or non-round wires will allow orienting the wire cross section such that it naturally causes the flaring motion upon deployment from the deployment tube. An example would be a rectangular wire with the long length of the cross-section oriented along the periphery of the device, around the heart. Furthermore, when engaging the deployable device with the self-expanding wire framework, the self-expanding wire fragment should be in a flaring position with the top segment 300 of each of the articulated wire loops out of plane.

In other embodiment of the invention, the midway bends of the articulated wire loop can be made more rounded to allow for gradual flaring as the framework is deployed. In another embodiment, the midway bends can also comprise multiple bends (two bends instead of one midway bend). Other embodiments can include 3, 4, 5 or more bends depending on the preference of the manufacturer. The diamond shape of the articulated wire loop is not the only possible element design. Various other shapes and designs are possible as long as there is a bending top segment 300 and midway bends in a left segment and a right segment that bend outward when the wire loop is collapsed and packed into the deployment tube.

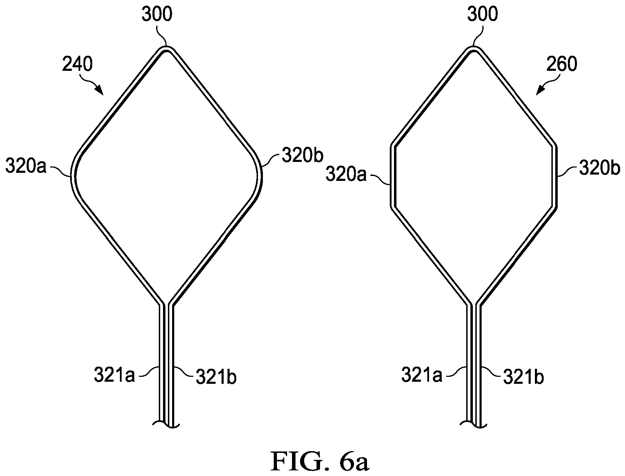

FIG. 6a shows a schematic of an embodiment of articulated wire loop 240 with the top segment 300 flanked by the left and right midway bends 320a and 320b which are more rounded and include strut 321a extending from midway bends 320a and strut 321b extending from midway bends 320b into the deployment tube (not shown). Another embodiment includes articulated wire loop 260 with the top segment 300 flanked by the left and right midway bends 320a and 320b which are more flat and include strut 321a extending from midway bends 320a and strut 321b extending from midway bends 320b into the deployment tube (not shown).

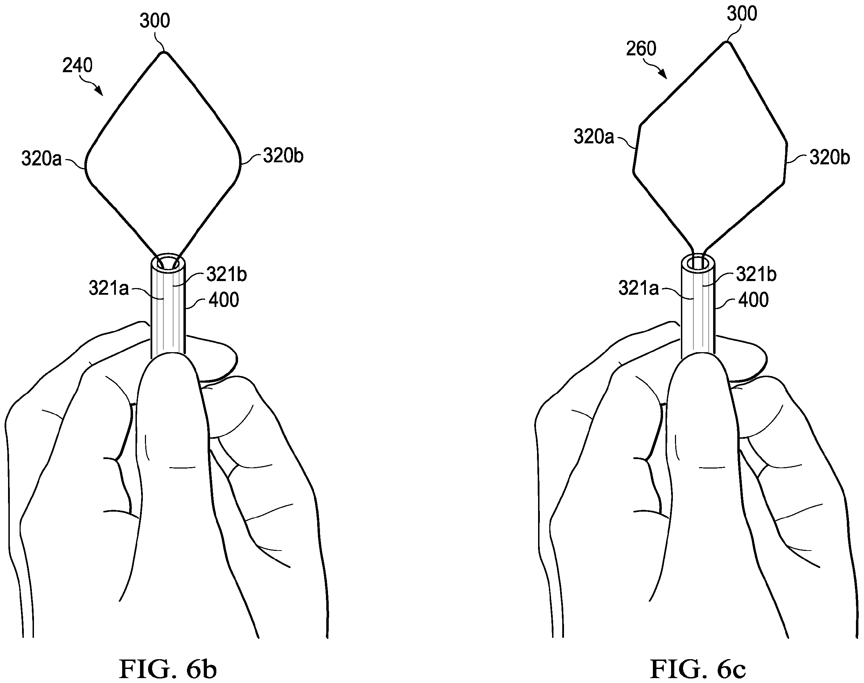

FIG. 6b shows an articulated wire loop 240 with the top segment 300 flanked by the left and right midway bends 320a and 320b which are more rounded and include strut 321a extending from midway bends 320a and strut 321b extending from midway bends 320b into the deployment tube 400.

FIG. 6c shows an articulated wire loop 260 with the top segment 300 flanked by the left and right midway bends 320a and 320b which are more flat and include strut 321a extending from midway bends 320a and strut 321b extending from midway bends 320b into the deployment tube 400.

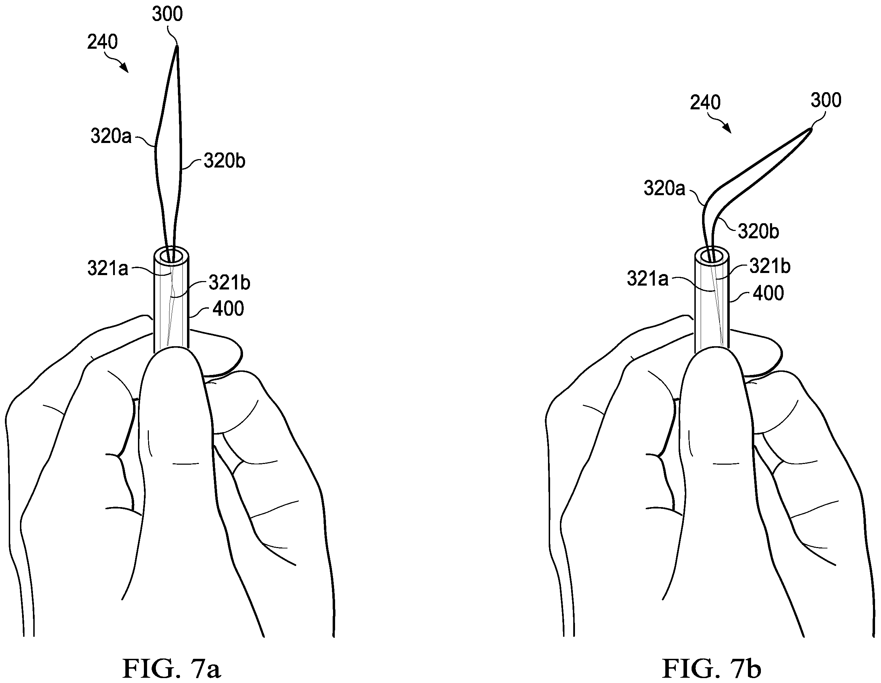

FIGS. 7a-7d illustrate a side view of articulated wire loop 240 being drawn into deployment tube 400.

FIG. 7a shows an articulated wire loop 240 with the top segment 300 flanked by the left and right midway bends 320a and 320b which are more rounded and include strut 321a extending from midway bends 320a and strut 321b extending from midway bends 320b drawn into the deployment tube 400.

FIG. 7b shows an articulated wire loop 240 with the top segment 300 flanked by the left and right midway bends 320a and 320b which are more rounded and include strut 321a extending from midway bends 320a and strut 321b extending from midway bends 320b drawn into the deployment tube 400.

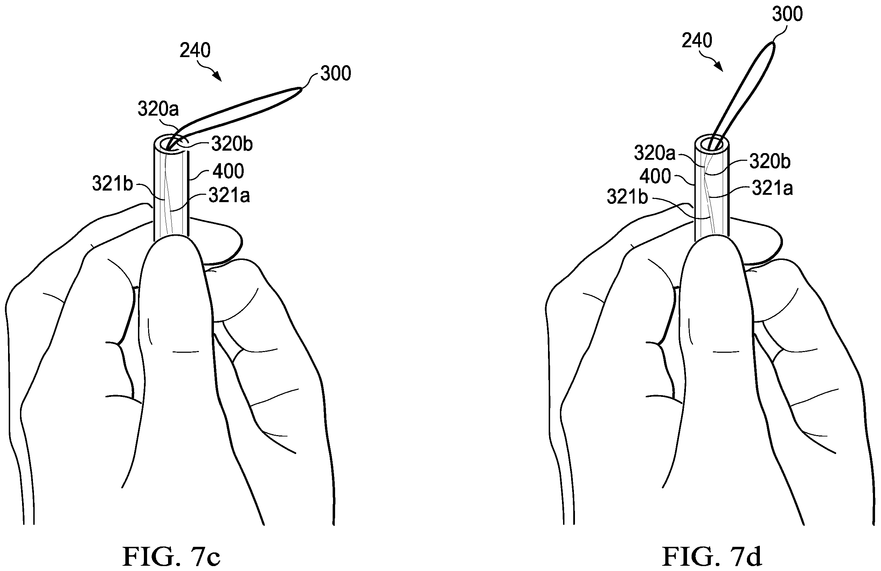

FIG. 7c shows an articulated wire loop 240 with the top segment 300 flanked by the left and right midway bends 320a and 320b which are more rounded and include strut 321a extending from midway bends 320a and strut 321b extending from midway bends 320b drawn into the deployment tube 400. The articulated wire loop 240 is drawn into the deployment tube 400 to the left and right midway bends 320a and 320b.

FIG. 7d shows an articulated wire loop 240 with the top segment 300 flanked by the left and right midway bends 320a and 320b which are more rounded and include strut 321a extending from midway bends 320a and strut 321b extending from midway bends 320b drawn into the deployment tube 400. The articulated wire loop 240 is drawn into the deployment tube 400 such that the left and right midway bends 320a and 320b are within the deployment tube 400.

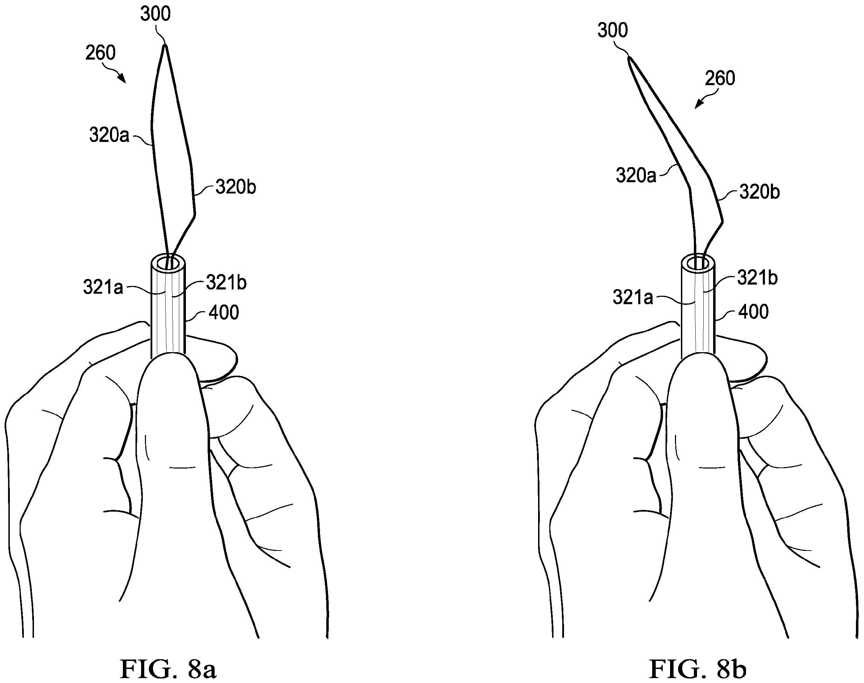

FIG. 8a shows an articulated wire loop 260 with the top segment 300 flanked by the left and right midway bends 320a and 320b which are more rounded and include strut 321a extending from midway bends 320a and strut 321b extending from midway bends 320b drawn into the deployment tube 400.

FIG. 8b shows an articulated wire loop 260 with the top segment 300 flanked by the left and right midway bends 320a and 320b which are more rounded and include strut 321a extending from midway bends 320a and strut 321b extending from midway bends 320b drawn into the deployment tube 400.

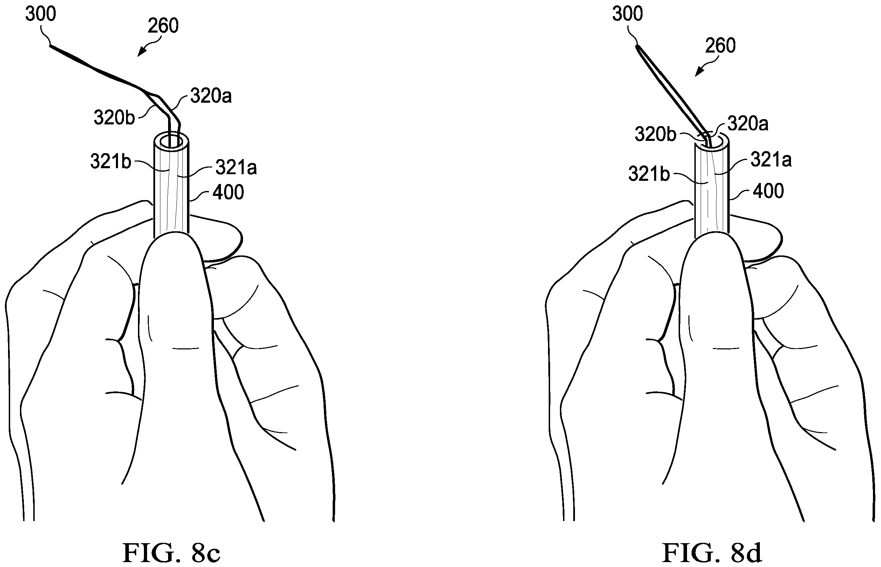

FIG. 8c shows an articulated wire loop 260 with the top segment 300 flanked by the left and right midway bends 320a and 320b which are more rounded and include strut 321a extending from midway bends 320a and strut 321b extending from midway bends 320b drawn into the deployment tube 400. The articulated wire loop 240 is drawn into the deployment tube 400 to the left and right midway bends 320a and 320b.

FIG. 8d shows an articulated wire loop 260 with the top segment 300 flanked by the left and right midway bends 320a and 320b which are more rounded and include strut 321a extending from midway bends 320a and strut 321b extending from midway bends 320b drawn into the deployment tube 400.

The articulated wire loop 240 is drawn into the deployment tube 400 such that the left and right midway bends 320a and 320b are within the deployment tube 400.

Various attachment means may also be utilized between a polymer film chamber and the wire frame so as not to impede on desired bending. One example as shown in FIGS. 3a-3e is to strap the wires between layers of plastic. Another embodiment would be to allow wire movement and rotation within the polymer film attachment points.

It will be understood that particular embodiments described herein are shown by way of illustration and not as limitations of the invention. The principal features of this invention can be employed in various embodiments without departing from the scope of the invention.

All publications and patent applications mentioned in the specification are indicative of the level of skill of those skilled in the art to which this invention pertains.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof' as used herein refers to all permutations and combinations of the listed items preceding the term. For example, "A, B, C, or combinations thereof' is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions without departing from the scope of the invention. All such similar substitutes and modifications apparent to those skilled in the art are deemed to be within the scope of the invention as defined by the appended claims.

## Claims

1. A self-expanding wire framework device (100) comprising:
a deployment tube (400) and
a self-expanding wire framework (200) positioned inside the deployment tube (400) and covered with a polymer film (221) adapted to flare outwardly to encircle a portion of a heart, wherein the self-expanding wire framework (200) comprises:
a set of adjacent articulated wire loops extending from a lead edge to a hub, **characterized in that** each of the articulated wire loops (200) of the set of articulated wire loops comprise:
a top segment (300) positioned at the lead edge and that extends to a left midway bend (320a) and to a right midway bend (320b),
a left strut (321a) that extends from the left midway bend (320a) to the hub,
a right strut (321b) that extends from the right midway bend (321b) to the hub, and
a fixed strut attachment point on the hub to fix the position of the left strut (321a) and the right strut (321b),
wherein each of the articulated wire loops (220) of the set of articulated wire loops is positioned to at least partially overlap an adjacent articulated wire loop, and
wherein the left midway bend (320a) and right midway bend (320b) result in a tension that causes the set of articulated wire loops to engage in a circumferential flaring motion and bend outwardly as the self-expanding framework (200) is deployed from the deployment tube (400) to outwardly flair the self-expanding wire framework (200) and the polymer film (221).

2. The self-expanding framework device (100) of claim 1, wherein the left midway bends (320a) and the right midway bends (320b) are rounded to allow for gradual flaring as the self-expanding wire framework (200) is deployed or wherein the left midway bends (320a) and the right midway bends (320a) are flattened to allow for flaring as the self-expanding wire framework (200) is deployed.

3. The self-expanding framework device (100) of claim 1 or 2, wherein the left midway bends (320a) and the right midway bends (320b) further comprise multiple bends to allow for immediate flaring positions as the self-expanding wire framework (200) is deployed.

4. The self-expanding framework device (100) of any of the claims 1 to 3, wherein the articulated wire loops (220) comprise flat wires, oval wires, round wires, non-rounded wires.

5. The self-expanding framework device (100) of any of the claims 1 to 4, where the set of articulated wire loops comprises at least 2, 3, 4, 5, 6, 7, 8, 9, 10, 11, 12, 13, 14, 15, 16, 17, 18, 19, 20 or more articulated wire loops (220).

6. The self-expanding framework device (100) of any of the claims 1 to 5, wherein the left strut (321a) is intertwined with the right strut (321b) between the midway bend and the hub.

7. The self-expanding framework device (100) of any of the claims 1 to 6, wherein the left strut (321a) and the right strut (321b) are connected separately at the hub.

8. The self-expanding framework device (100) of any of the claims 1 to 7, wherein each of the articulated wire loops (220) of the set of articulated wire loops is positioned adjacent to an adjacent articulated wire loop (220).

9. The self-expanding framework device (100) of any of the claims 1 to 8, wherein the polymer film (221) is on the inside of the self-expanding wire framework (200), on the outside of the self-expanding wire framework (200) or on the inside and the outside to sandwich the self-expanding wire framework (200).

10. The self-expanding framework device (100) of any of the claims 1 to 9, wherein the left strut (321a) and the right strut (321b) each comprise a bend end connected to the midway bend and a hub end, wherein the hub end is secured to the hub at a fixed strut attachment point.

11. The self-expanding framework device (100) of any of the claims 1 to 10, wherein the deployment tube (400) having an outer surface surrounding an inner passage and a deployment aperture at one end of the inner passage, wherein the self-expanding wire framework (200) covered with a polymer film (221) is slidably positioned in the inner passage to slidably extend from the deployment aperture and adapted to flare outwardly to encircle a larger portion of the heart.

## Patentansprüche

1. Eine selbstexpandierende Drahtrahmenvorrichtung (100), aufweisend:
ein Einsatzrohr (400) und
einen selbstexpandierenden Drahtrahmen (200), der innerhalb des Einsatzrohrs (400) positioniert ist und mit einer Polymerfolie (221) bedeckt ist, die so angepasst ist, dass sie sich nach außen ausdehnt, um einen Teil eines Herzes zu umschließen, wobei der selbstexpandierende Drahtrahmen (200) aufweist:
eine Reihe benachbarter gelenkiger Drahtschleifen, die sich von einer Vorderkante zu einer Nabe erstrecken, **dadurch gekennzeichnet, dass** jede der gelenkigen Drahtschleifen (200) der Reihe von gelenkigen Drahtschleifen aufweist:
ein oberes Segment (300), das an der Vorderkante positioniert ist und sich zu einer linken Mittelbiegung (320a) und zu einer rechten Mittelbiegung (320b) erstreckt,
eine linke Strebe (321a), die sich von der linken Mittelbiegung (320a) zur Nabe erstreckt,
eine rechte Strebe (321b), die sich von der rechten Mittelbiegung (320b) zur Nabe erstreckt, und
einen festen Strebenbefestigungspunkt an der Nabe, um die Position der linken Strebe (321a) und der rechten Strebe (321b) zu fixieren,
wobei jede der gelenkigen Drahtschleifen (220) der Reihe von gelenkigen Drahtschleifen so positioniert ist, dass sie zumindest teilweise eine benachbarte gelenkige Drahtschleife überlappt, und
wobei die linke Mittelbiegung (320a) und die rechte Mittelbiegung (320b) zu einer Spannung führen, die bewirkt, dass die Reihe von gelenkigen Drahtschleifen eine umfangsmäßige Ausweitung ausführt und sich nach außen biegt, wenn der selbstexpandierende Rahmen (200) aus dem Einsatzrohr (400) eingesetzt wird, um den selbstexpandierenden Drahtrahmen (200) und die Polymerfolie (221) nach außen aufzufächern.

2. Die selbstexpandierende Rahmenvorrichtung (100) nach Anspruch 1, wobei die linken Mittelbiegungen (320a) und die rechten Mittelbiegungen (320b) abgerundet sind, um eine allmähliche Ausweitung zu ermöglichen, wenn der selbstexpandierende Drahtrahmen (200) eingesetzt wird, oder wobei die linken Mittelbiegungen (320a) und die rechten Mittelbiegungen (320a) abgeflacht sind, um eine Ausweitung zu ermöglichen, wenn der selbstexpandierende Drahtrahmen (200) eingesetzt wird.

3. Die selbstexpandierende Rahmenvorrichtung (100) nach Anspruch 1 oder 2, wobei die linken Mittelbiegungen (320a) und die rechten Mittelbiegungen (320b) weitere Mehrfachbiegungen aufweisen, um sofortige Ausweitungspositionen zu ermöglichen, wenn der selbstexpandierende Drahtrahmen (200) eingesetzt wird.

4. Die selbstexpandierende Rahmenvorrichtung (100) nach einem der Ansprüche 1 bis 3, wobei die gelenkigen Drahtschleifen (220) flache Drähte, ovale Drähte, runde Drähte, nicht-runde Drähte aufweisen.

5. Die selbstexpandierende Rahmenvorrichtung (100) nach einem der Ansprüche 1 bis 4, wobei die Reihe von gelenkigen Drahtschleifen mindestens 2, 3, 4, 5, 6, 7, 8, 9, 10, 11, 12, 13, 14, 15, 16, 17, 18, 19, 20 oder mehr gelenkige Drahtschleifen (220) aufweist.

6. Die selbstexpandierende Rahmenvorrichtung (100) nach einem der Ansprüche 1 bis 5, wobei die linke Strebe (321a) zwischen der Mittelbiegung und der Nabe mit der rechten Strebe (321b) verflochten ist.

7. Die selbstexpandierende Rahmenvorrichtung (100) nach einem der Ansprüche 1 bis 6, wobei die linke Strebe (321a) und die rechte Strebe (321b) separat an der Nabe verbunden sind.

8. Die selbstexpandierende Rahmenvorrichtung (100) nach einem der Ansprüche 1 bis 7, wobei jede der gelenkigen Drahtschleifen (220) der Reihe von gelenkigen Drahtschleifen neben einer benachbarten gelenkigen Drahtschleife (220) positioniert ist.

9. Die selbstexpandierende Rahmenvorrichtung (100) nach einem der Ansprüche 1 bis 8, wobei die Polymerfolie (221) auf der Innenseite des selbstexpandierenden Drahtrahmens (200), auf der Außenseite des selbstexpandierenden Drahtrahmens (200) oder auf der Innen- und Außenseite angeordnet ist, um den selbstexpandierenden Drahtrahmen (200) einzuschließen.

10. Die selbstexpandierende Rahmenvorrichtung (100) nach einem der Ansprüche 1 bis 9, wobei die linke Strebe (321a) und die rechte Strebe (321b) jeweils ein Biegeende aufweisen, das mit der Mittelbiegung verbunden ist, und ein Nabenende, wobei das Nabenende an der Nabe an einem festen Strebenbefestigungspunkt befestigt ist.

11. Die selbstexpandierende Rahmenvorrichtung (100) nach einem der Ansprüche 1 bis 10, wobei das Einsatzrohr (400) eine äußere Oberfläche aufweist, die einen inneren Durchgang umgibt, und eine Einsatzöffnung an einem Ende des inneren Durchgangs aufweist, wobei der mit einer Polymerfolie (221) bedeckte selbstexpandierende Drahtrahmen (200) verschiebbar im inneren Durchgang positioniert ist, um verschiebbar aus der Einsatzöffnung herauszutreten und so angepasst ist, dass er sich nach außen ausweitet, um einen größeren Teil des Herzens zu umschließen.

## Revendications

1. Dispositif de cadre métallique auto-expansible (100) comprenant :
un tube de déploiement (400), et
un cadre métallique auto-expansible (200) positionné à l'intérieur du tube de déploiement (400) et recouvert avec un film polymère (221) adapté pour s'évaser vers l'extérieur afin d'encercler une partie d'un coeur, dans lequel le cadre métallique auto-expansible (200) comprend :
un ensemble de boucles de fil articulées adjacentes s'étendant à partir d'un bord avant jusqu'à un moyeu, **caractérisé en ce que** chacune des boucles de fil articulées (200) de l'ensemble de boucles de fil articulées comprend :
un segment supérieur (300) positionné au niveau du bord avant et qui s'étend vers un coude médian gauche (320a) et un coude médian droit (320b),
une entretoise gauche (321a) qui s'étend du coude médian gauche (320a) au moyeu,
une entretoise droite (321b) qui s'étend du coude médian droit (321b) au moyeu, et
un point de fixation d'entretoise fixe sur le moyeu pour déterminer la position de l'entretoise gauche (321a) et de l'entretoise droite (321b),
dans lequel chacune des boucles de fil articulées (220) de l'ensemble de boucles de fil articulées est positionnée pour chevaucher au moins partiellement une boucle de fil articulée adjacente, et
dans lequel le coude médian gauche (320a) et le coude médian droit (320b) se traduisent par une tension qui amène l'ensemble de boucles de fil articulées à se mettre en prise dans un mouvement d'évasement circonférentiel et à se courber vers l'extérieur lorsque le cadre auto-expansible (200) est déployé à partir du tuyau de déploiement (400) pour évaser vers l'extérieur du cadre métallique auto-expansible (200) et le film polymère (221).

2. Dispositif de cadre métallique auto-expansible (100) selon la revendication 1, dans lequel les coudes médians gauches (320a) et les coudes médians droits (320b) sont arrondis pour permettre l'évasement progressif lorsque le cadre métallique auto-expansible (200) est déployé ou bien dans lequel les coudes médians gauches (320a) et les coudes médians droits (320a) sont aplatis pour permettre l'évasement lorsque le cadre métallique auto-expansible (200) est déployé.

3. Dispositif de cadre métallique auto-expansible (100) selon la revendication 1 ou 2, dans lequel les coudes médians gauches (320a) et les coudes médians droits (320b) comprennent en outre plusieurs coudes pour permettre des positions d'évasement immédiates lorsque le cadre métallique auto-expansible (200) est déployé.

4. Dispositif de cadre métallique auto-expansible (100) selon l'une quelconque des revendications 1 à 3, dans lequel les boucles de fil articulées (220) comprennent des fils plats, des fils ovales, des fils ronds, des fils non arrondis.

5. Dispositif de cadre métallique auto-expansible (100) selon l'une quelconque des revendications 1 à 4, dans lequel l'ensemble de boucles de fil articulées comprend au moins 2, 3, 4, 5, 6, 7, 8, 9, 10, 11, 12, 13, 14, 15, 16, 17, 18, 19, 20 boucles de fil articulées (220) ou plus.

6. Dispositif de cadre métallique auto-expansible (100) selon l'une quelconque des revendications 1 à 5, dans lequel l'entretoise gauche (321a) est entrelacée avec l'entretoise droite (321b) entre le coude médian et le moyeu.

7. Dispositif de cadre métallique auto-expansible (100) selon l'une quelconque des revendications 1 à 6, dans lequel l'entretoise gauche (321a) et l'entretoise droite (321b) sont raccordées séparément au niveau du moyeu.

8. Dispositif de cadre métallique auto-expansible (100) selon l'une quelconque des revendications 1 à 7, dans lequel chacune des boucles de fil articulées (220) de l'ensemble de boucles de fil articulées est positionnée de manière adjacente à une boucle de fil articulée (220) adjacente.

9. Dispositif de cadre métallique auto-expansible (100) selon l'une quelconque des revendications 1 à 8, dans lequel le film polymère (221) est à l'intérieur du cadre métallique auto-expansible (200), à l'extérieur du cadre métallique auto-expansible (200) ou à l'intérieur et à l'extérieur pour prendre en sandwich le cadre métallique auto-expansible (200).

10. Dispositif de cadre métallique auto-expansible (100) selon l'une quelconque des revendications 1 à 9, dans lequel l'entretoise gauche (321a) et l'entretoise droite (321b) comprennent chacune une extrémité de coude raccordée au coude médian et à une extrémité de moyeu, dans lequel l'extrémité de moyeu est fixée au moyeu au niveau d'un point de fixation d'entretoise fixe.

11. Dispositif de cadre métallique auto-expansible (100) selon l'une quelconque des revendications 1 à 10, dans lequel le tube de déploiement (400) ayant une surface externe entourant un passage interne et une ouverture de déploiement au niveau d'une extrémité du passage interne, dans lequel le cadre métallique auto-expansible (200) recouvert avec un film polymère (221) est positionné, de manière coulissante, dans le passage interne pour s'étendre de manière coulissante, à partir de l'ouverture de déploiement et adapté pour s'évaser vers l'extérieur afin d'encercle une plus grande partie du coeur.
